# EUROPEAN PATENT SPECIFICATION

(11) **EP 1 190 706 B1**
(45) Date of publication and mention of the grant of the patent: **06.05.2009**
(21) Application number: 00939165.7
(22) Date of filing: 26.06.2000
(51) Int. Cl.: A61K 9/127, A61K 47/18

(54) **LIPOSOMES**
LIPOSOME
LIPOSOMES

(30) Priority: 25.06.1999 JP 18015899
(43) Date of publication of application: 27.03.2002
(73) Proprietor: TERUMO KABUSHIKI KAISHA, Tokyo 151-0072 (JP)
(72) Inventor: KONDO, Masayo Terumo Kabushiki Kaisha, Ashigarakami-gun, Kanagawa 259-0151 (JP); HARIGAE, Takashi Terumo Kabushiki Kaisha, Ashigarakami-gun, Kanagawa 259-0151 (JP); KIMURA, Junji Terumo Kabushiki Kaisha, Ashigarakami-gun, Kanagawa 259-0151 (JP); ISOZAKI, Masashi Terumo Kabushiki Kaisha, Ashigarakami-gun, Kanagawa 259-0151 (JP)
(74) Representative: Nevant, Marc
(86) International application number: PCT/JP2000/004182
(87) International publication number: WO 2001/000174

(56) References cited:
- EP-A1- 1 044 679
- WO-A-00/25748
- WO-A-97/42166
- JP-A- 7 145 038
- JP-A- 8 027 030
- JP-A- 9 263 579
- JP-A- 10 120 595
- MILLER C R ET AL: "LIPOSOME-CELL INTERACTIONS IN VITRO: EFFECT OF LIPOSOME SURFACE CHARGE ON THE BINDING AND ENDOCYTOSIS OF CONVENTIONAL AND STERICALLY STABILIZED LIPOSOMES" BIOCHEMISTRY, AMERICAN CHEMICAL SOCIETY. EASTON, PA, US, vol. 37, no. 37, 1998, pages 12875-12883, XP000929364 ISSN: 0006-2960

## Description

### TECHNICAL FIELD

The present invention relates to a liposome including a drug.

### BACKGROUND ART

In recent years, there have been increasing studies on drug delivery system (DDS) that can perform unfailingly, efficiently and safely targeting a drug, DNA, peptide and protein to an objective site, i.e., a diseased site, for example, cancer, pneumonia, hepatitis, nephritis, lymphoma, injured site of vascular endothelia or the like.

There may be cited, for example, a method in which closed vesicles such as liposome, emulsions, lipid microspheres, and nano particles are utilized as a drug carrier, a method in which a drug is included in or bound to a polymer carrier such as high molecular synthetic polymer micelle or polysaccharide, a method in which such a closed vesicle or a polymer carrier is modified on its surface with a high molecular functional molecule such as antibody or protein, or a low molecular functional molecule such as a specified sugar chain, peptide or the like to increase target directivity, and so forth. However, there are various problems to be overcome in practical use of these drug carriers. Among them, difficulty of evasion from the foreign matter recognizing mechanism of organism and control of pharmacokinetics in the body cause problems. In particular, closed vesicles have been difficult to perform delivery to a targeted tissue or cell with high selectivity due to its aggregation by the interaction with opsonin protein or plasma protein in blood or capture in reticuloendothelial system tissue (RES) such as liver or spleen.

As a means for solving this problem, it has been made possible to prevent adsorption of plasma protein, opsonin protein or the like to increase the stability in blood to evade capture in the RES by coating the surface of a polymer carrier inclusive of the closed vesicles with a hydrophilic polymer such as polyethylene glycol (PEG). Closed vesicles such as liposome are passively accumulated in a tissue in which the vascular permeability has been augmented in tumor tissues or at inflammatory sites because they can provide long residence in blood. This makes it possible to perform efficient treatment. On the other hand, in the case where the closed vesicles have acquired properties of evading RES on the basis of the above-mentioned modification, the interaction between liposome and the target tissue is decreased. This improves the delivery of the closed vesicles to the target tissue. On the contrary, this causes defects in that the efficiency of incorporation of the closed vesicles by the cells in the target tissue is decreased to thereby decrease the efficiency of delivery of a drug enclosed in the closed vesicles to the cells, or the disintegration of liposome caused by the interaction with a biocomponent such as protein in blood is inhibited, with the result that the release rate of the drug enclosed in the closed vesicles is decreased.

Many liposomes comprising basic compounds as membrane components have hitherto been studied as tools for transporting genes into cells. Liposomes comprising various basic compounds in the membranes have affinity for substances having a negative charge. In particular, the cell surface is constituted by a substance having a negative charge like a sulfated polysaccharide such as heparan sulfate and hence liposomes comprising basic compounds are expected to have strong affinity for the cell membrane. However, such a liposome comprising a basic compound when it is administered in blood binds to a substance having a negative charge such as protein in serum or the surface of erythrocyte membrane, so that the surface charge is changed or aggregation in blood is caused and loss of affinity for the cell membrane is concerned about. Therefore, a drug delivery system is desired that circulates stably having no interaction with protein or cells in blood flow and has increased interaction with cells only in a diseased site so that it can deliver the drug to the cells.

To solve these problems, various environment responsive drug carriers, that is, drug carriers that change properties in response to a change in environment due to diseases, or a difference in environment between a normal tissue and a diseased site have been studied. For example, it is known that at a diseased site such as tumor or inflammation, local pH is lowered as compared with normal tissue (V. Menkin, Biochemical Mechanism in Inflammation, Thomas, Springfield, III, pp.69-7 (1956)) and studies.have been approached directed to releasing or targeting a drug in response to an environment due to a pH change at such a lesion site (Biochim Biophys Acta, 1329(2), 291-301 (1997); FEBS, 421(1), 61-4 (1998)).

EP 1 044 679, constituting a prior art under Article 54(3) EPC, discloses a liposome having a drug included therein, said liposome having a targeting property and ensured stability in blood. The liposome's membrane comprises (1) a basic compound that takes positive charge within a physiological pH range, (2) a lipid derivative of hydrophilic polymer, (3) a lipid, which constitutes the liposome and (4) a stabilising agent such as sterol. The liposome of EP 1 044 679 can be used effectively in the diagnosis and/or therapy.

Furthermore, WO 97/42166 discloses a liposome as a drug carrier, wherein said liposome comprises as membrane constituents: (1) amidine derivative as a basic compound, (2) a phospholipid as a lipid constituting the liposome and (3) a stabilising agent such as sterol.

However, no report has been made yet on a liposome that responds to a change in pH, improving target directivity thereof.

### DISCLOSURE OF THE INVENTION

The present invention has been made to overcome the defects of conventional target directive liposome and provide a target directive liposome that responds to any environmental change. That is, an object of the present invention is to provide a liposome that exhibits target directive capability for a drug, DNA, peptide and protein by a change in pH at a diseased site.

The liposome of the present invention under such a physiological pH condition as in circulating blood weakly interacts with a substance having a negative charge, and hence it interacts with cells and proteins less frequently, whereby it is stable also in blood. However, at a target site where pH is decreased such as an inflammatory site or a tumor site, its interaction with a substance having a negative charge is enhanced, and thus its affinity for cells is improved, and it is efficiently accumulated at the target site along with that its accumulation outside the target site is prevented. As a result, an effect of alleviation of side effects can be expected.

The above-mentioned problems can be solved by the present invention as described below.
(1) The present invention relates to a liposome that includes a drug intended for the therapy and/or diagnosis, comprising as membrane constituents:
   [1] a basic compound represented by the following Formula 1: 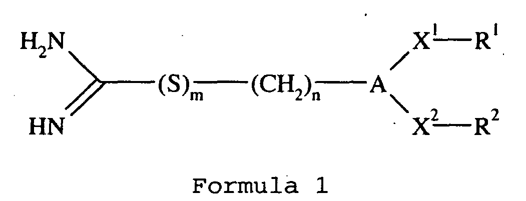 wherein in Formula 1 A represents an aromatic ring; R¹ and R² represent an alkyl group or alkenyl group having 10 to 25 carbon atoms, where R¹ and R² may be the same or different; X¹ and X² represent -O-, -S-, -COO-, -OCO-, -CONH- or NHCO-, where X¹ and X² may be the same or different; m is 0 or 1, and n is 0 or an integer of 1 to 6;
   [2] an acidic compound selected from a phosphoric acid monoester derivative, or a compound having a carboxyl group or its salt, said phosphoric acid monoester derivative being selected from prednisolone phosphate and riboflavin phosphate and the molar ratio of said acidic compound, which is a phosphoric acid monoester derivative or a carboxylic acid derivative, is 0.5 to 30 mol% of total liposome membrane constituents;
   [3] a liposome membrane constituent other than [1] and [2].
(2) The present invention relates to a liposome as defined above that is efficiently accumulated at a diseased site such as an inflammatory site or a tumor site in an environment of pH 5 to 7.
(3) The present invention relates to the liposome according to the above item (1), in which a molar ratio of the basic compound is 1 to 30 mol% of total liposome membrane constituents.
(4) The present invention relates to the liposome according to the above item (1), in which the acidic compound, which is a compound having a carboxyl group or its salt, is a fatty acid.
(5) The present invention relates to the liposome according to the above item (4), in which the fatty acid is oleic acid, stearic acid, palmitic acid, or myristic acid.
(6) The present invention relates to the liposome according to the above item (1), in which the other liposome membrane constituent is phospholipid and/or sterol.
(7) The present invention relates to the liposome according to the above item (1), in which the drug intended for the therapy and/or diagnosis is an anti-cancer agent, an antibiotic, an enzyme agent, an enzyme inhibitor, an antioxidant, a lipid uptake inhibitor, a hormone agent, an anti-inflammatory agent, a steroid agent, a vasodilator, an angiotensin converting enzyme inhibitor, an angiotensin receptor antagonist, a growth/migration inhibitor for smooth muscle cells, a platelet aggregation inhibitor, an anticoagulant, a chemical mediator releasing inhibitor, a vascular endothelial cell growth or suppressing agent, an aldose reductase inhibitor, a mesangium cell growth inhibitor, a lipoxygenase inhibitor, an immunosuppressor, an immunoactivator, an antiviral agent, a Maillard reaction inhibitor, an amyloidosis inhibitor, an NOS inhibitor, an AGEs inhibitor, or a radical scavenger.
(8) The present invention relates to the liposome according to the above item (1), in which the drug intended for the therapy and/or diagnosis is a nucleic acid, a polynucleotide or a gene.
(9) The present invention relates to the liposome according to the above item (1), in which the drug intended for the therapy and/or diagnosis is glycosaminoglycan.
(10) The present invention relates to the liposome according to the above item (1), in which the drug for the therapy and/or diagnosis is oligo- and/or polysaccharide.
(11) The present invention relates to the liposome according to the above item (1), in which the drug intended for the therapy and/or diagnosis is protein or peptide.
(12) The present invention relates to the liposome according to the above item (1), in which the drug intended for the therapy and/or diagnosis is an intracorporeal diagnostic drug such as an X-ray contrast medium, a radiolabeled nuclear medicinal diagnostic drug or a nuclear magnetic resonance diagnostic drug for diagnosis.
(13) The liposome according to the invention is useful for increasing the ratio of accumulation of liposome at a diseased site, such that a ratio of an adsorption amount of chondroitin sulfate C to liposome in a phosphate buffer of pH 6.5 to that in a phosphate buffer of pH 7.4 is at least 1.5.

To the liposome of the present invention, a lipid derivative of a hydrophilic polymer as a surface modifying agent may be added as needed, and its molecular weight is desirably 1,000 to 7,000. As the lipid derivative of the hydrophilic polymer, polyethylene glycol derivatives are desirable. In particular, compounds having a polyethylene glycol chain and diacyl glycerol in one molecule are desirable.

### BRIEF DESCRIPTION OF DRAWINGS

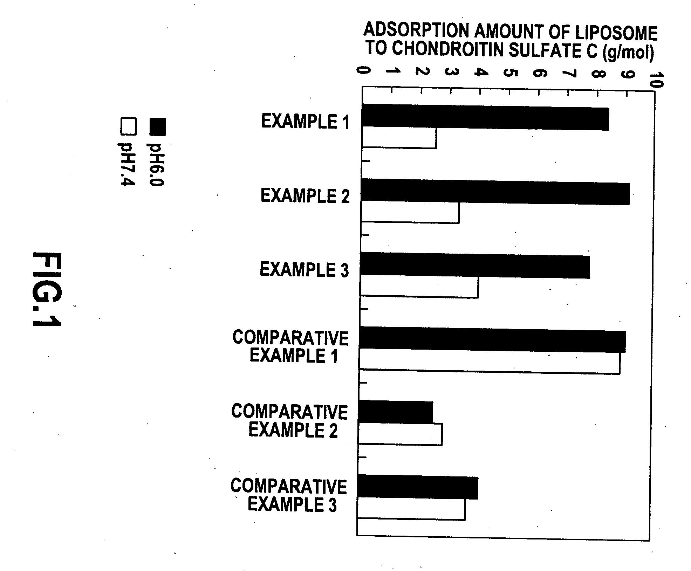

Fig. 1 is a graph illustrating affinity of each liposome to proteoglycan (chondroitin sulfate C).

### BEST MODE FOR CARRYING OUT THE INVENTION

Hereinafter, explanation will be made on embodiments of the present invention. The constituent of the liposome of the present invention is composed of a basic compound that gives to a membrane a positive charge from at near physiological pH to acidic conditions when it is embedded in the membrane by itself, an acidic compound that has a negative charge at physiological pH but ionization of which is suppressed to decrease a negative charge at near physiological pH to acidic side when it is embedded into the membrane by itself, and another liposome constituent components. It is not particularly limited and can be used as far as it forms a microspheric structure and has safety.

Here, the physiological pH condition means the pH in the living body, that is, around 7.4, approximately in the range of 7.2 to 7.6. The acidic side than near physiological pH indicates the acidic side than near pH 7.4, that is, the acidic side approximately at a pH in the range of 6 to 8. The acidic compounds, whose dissociation is prevented from this region, are expected to exhibit the performance due to a combination with the basic compound. However, compounds whose dissociation is prevented only in extremely acidic region such as pH 4 or less, for example, phosphoric acid diester derivatives, do not have such an action and hence are not included in the acidic compound of the present invention as will be apparent from the examples described hereinbelow.

Specific examples of the constituent of liposome membrane of the present invention include the basic compound represented by Formula 1 above, an acidic compound, which is a phosphoric acid monoester derivative as defined above or a compound having a carboxyl group or its salt, and another liposome membrane constituent, and it is not particularly limited and can be used as far as it forms a microspheric structure and has safety.

The amount of the basic compound is preferably 1 to 30, further 5 to 30 mol% of the total liposome membrane constituent. In this range, the accumulation to a diseased site such as an inflammatory site or a tumor site at a pH of 5 to 7 is increased.

The phosphoric acid monoester derivative is selected among prednisolone phosphate and riboflavin phosphate. In particular, the acidic compound, which is phosphoric acid monoester derivative is desirable as a constituent of the liposome of the present invention since it is specifically accumulated at a diseased site such as an inflammatory site or a tumor site in the region of acidic pH.

The compound having a carboxyl group or its salt is a compound having a -COOX⁸ group, where X⁸ represents hydrogen or a pharmaceutically acceptable cation such as sodium or potassium. A fatty acid is not particularly limited unless the structural stability of liposome is deteriorated. Specifically, there can be cited natural or synthetic oleic acid, palmitic acid, stearic acid, myristic acid and linolenic acid.

The amount of the acidic compound, which is a phosphoric monoester derivative or a carboxylic acid derivative, is preferably 0.5 to 30 mol% of the total liposome membrane constituent. In this range, accumulation at a diseased site such as an inflammatory site or a tumor site at a pH of 5 to 7 is increased.

The present invention is characterized by adding in addition to [1] a basic compound, [2] a specified acidic compound as liposome membrane constituent. Phosphatidylcholine that has been conventionally used as a lipid is a phosphoric acid diester compound. However, this is a dipolar ionic compound having an acidic group and a basic group in one molecule and is neutral at near physiological pH.

As the other liposome membrane constituents excluding the above-mentioned basic compound and the above-mentioned acidic compound, there can be cited phospholipids and sterols such as cholesterol. As the phospholipid, there can be cited natural or synthetic phospholipids, for example, phosphatidylcholine (lecithin), phosphatidylglycerol, phosphatidylethanolamine, phasphatidylserine, phosphatidylinositol, sphingomyelin, cardiolipin, and those obtained by hydrogenating these by a common method.

The liposome membrane of the present invention may further contain a stabilizer, which includes, for example, sterol such as cholesterol, and sugars such as glycerol and sucrose that decrease membrane fluidity. As an antioxidant, there can be cited, for example, tocopherol homologues, that is, vitamin E. Tocopherol exists as four isomers α, β, γ and δ. Any one may be used in the present invention.

A lipid derivative of a hydrophilic polymer that modifies the liposome surface is not particularly limited unless it deteriorates the structural stability of liposome. There can be cited, for example, polyethylene glycol, dextran, pullulan, ficoll, polyvinyl alcohol, synthetic polyamino acid, amylose, amylopectin, mannan, cyclodextrin, pectin, carrageenan, and derivatives thereof. Among them, polyethylene glycol and polyethylene glycol derivatives are desirable.

The molecular weight of the lipid derivative of the hydrophilic polymer is preferably 1,000 to 7,000. In particular, polyethylene glycol and polyethylene glycol derivatives having a molecular weight of 1,000 and 7,000 have considerable effect of improving retention in blood and thus are preferred. The polyethylene glycol derivative is not particularly limited. However, it is preferably a compound having a polyethylene glycol chain and a diacyl glycerol in one molecule.

To modify the liposome surface with the hydrophilic polymer, various methods may be cited and not particularly limited. However, a method in which the above-mentioned hydrophilic polymer is bound to a hydrophobic compound such as a long-chain aliphatic alcohol, sterol, a polyoxypropylene alkyl, a glycerol fatty acid ester or a phospholipid and the part of the hydrophobic compound is inserted into the liposome surface is preferred.

As a drug for the diagnosis and/or therapy, pharmaceutically acceptable pharmacologically active substance, physiologically active substance and/or substance for diagnosis may be used depending on the purpose. The kind of drug is not particularly limited unless the formation of liposome is not damaged.

Specifically, there may be cited an anti-cancer agent, an antibiotic, an enzyme agent, an antioxidant, a lipid uptake inhibitor, a hormone agent, an anti-inflammatory agent, a steroid agent, a vasodilator, an angiotensin converting enzyme inhibitor, an angiotensin receptor antagonist, a growth/migration inhibitor for smooth muscle cells, a platelet aggregation inhibitor, an anticoagulant, a chemical mediator releasing inhibitor, a vascular endothelial cell growth accelerating or suppressing agent, an aldose reductase inhibitor, a mesangium cell growth inhibitor, a lipoxygenase inhibitor, an immunosuppressor, an immunoactivator, an antiviral agent, a Maillard reaction inhibitor, an amyloidosis inhibitor, a nitrogen monoxide synthesis inhibitor, an advanced glycation endproducts inhibitor, a radical scavenger, a protein, a peptide, a nucleic acid, a polynucleotide and a gene.

For example, as the examples of anti-cancer agent, there can be cited cyclophosphamide, ifosfamide, nitrogen mustard N-oxide hydrochloride, thiotepa, busulfan, carboquone, nimustine hydrochloride, ranimustine, melphalan, improsulfan tosylate, dacarbazine, procarbazine hydrochloride, cytarabine, cytarabine oxfate, enocitabine, mercaptopurine, thioinosine, fluorouracil, doxifluridine, tegafur, methotrexate, carmofur, hydroxycarbamide, vincristine sulfate, vinblastine sulfate, vindesine sulfte, etoposide, cromomycin A₃, daunorubicin hydrochloride, doxorubicin hydrochloride, aclarubicin hyrochloride, pirarubicin, epirubicin hydrochloride, dactinomycin, mitoxantrone hydrochloride, bleomycin hydrochloride, peplomycin sulfate, mitomycin C, neocarzinostatin, L-asparaginase, aceglatone mitobronitol, sodium dextran sulfate, octreotide acetate, cisplatin, carboplatin, tamoxifen citrate, medroxyprogesterone acetate, sodium estramustine phosphate, goserelin acetate, leuprorelin acetate and irinotecan hydrochloride.

As the antibiotics, there can be cited benzylpenicillin potassium, benzylpenicillin benzathine, phenoxymethylpenicillin potassium, phenethicillin potassium, cloxacillin sodium, flucloxacillin sodium, ampicillin, sultamicillin tosylate, bacampicillin hydrochloride, talampicillin hydrochloride, lenampicillin, hetacillin potassium, ciclacillin, amoxicillin, pivmecillinam hydrochloride, aspoxicillin, carbenicillin sodium, carindacillin sodium, sulbenicillin sodium, ticarcillin sodium, piperacillin sodium, cephaloridine, cephalotin sodium, cefazolin sodium, cefapirin sodium, cefradine, cephalexin, cefatrizine propylene glycol, cefroxadine, cefaclor, cefadroxil, cefotiam hydrochloride, cefotiam hexetil hydrochloride, cefuroxime sodium, cefuroxime axetil, cefamandole sodium, cefdinir, cefetamet pivoxil hydrochloride, ceftibuten, cefmetazole sodium, cefoxitin sodium, cefotetan sodium, cefminox sodium, cefbuperazone sodium, cefpiramide sodium, cefsulodin sodium, cefotaxime sodium, cefoperazone sodium, ceftizoxime sodium, cefmenoxime hydrochloride, ceftriaxone sodium, ceftazidime, sodium cefpimizole, cefixime, cefteram pivoxil, cefuzonam sodium, cefpodoxim proxetil, cefodizime, cefpirome sulfate, latamoxef sodium, flomoxef sodium, imipenem, cilastatin sodium, aztreonam, carumonam sodium, streptomycin sulfate, kanamycin sulfate, fradiomycin sulfate, amikacin sulfate, gentamicin sulfate, paromomycin sulfate, bekanamycin sulfate, ribostamycin sulfate, dibekacin sulfate, tobramycin, sisomicin sulfate, micronomicin sulfate, astromicin sulfate, netilmicin sulfate, isepamicin sulfate, arbekacin sulfate, erythromycin, kitasamycin, acetylkitasamycin, oleandomycin phosphate, josamycin, acetylspiramycin, midecamycin, midecamycin acetate, rokitamycin, roxithromycin, clarithromycin, tetracycline hydrochloride, oxytetracycline hydrochloride, tetracycline metaphosphate, demethylchlortetracycline hydrochloride, rolitetracycline, doxycycline hydrochloride, minocycline hydrochloride, chloramphenicol, chloramphenicol succinate sodium, chloramphenicol palmitate, thiamphenicol, thiamphenicol aminoacetate hydrochloride, colistin sulfate, colistin methanesulfonate sodium, polymxin B sulfate, bacitracin, vancomycin hydrochloride, lincomycin hydrochloride, clindamycin, spectinomycin hydrochloride, fosfomycin sodium and fosfomycin calcium.

As examples of the enzyme agent, there can be cited chymotrypsin, crystalline trypsin, streptokinase, streptodornase, hyaluronidase, urokinase, nasaruplase, alteplase, lysozyme, chloride, semialkaline protinase, serrapeptase, tisokinase, duteplase, batroxobin, pronase and promeline.

As examples of the antioxidant, there can be cited tocopherol, ascorbic acid and uric acid.

As examples of the anti-inflammatory agent, there can be cited choline salicylate, sasapyrine, sodium salicylate, aspirin, diflunisal, frufenamic acid, mefenamic acid, floctafenine, tolfenamic acid, diclofenac sodium, tolmetin sodium, sulindac, fenbufen, felbinac ethyl, indomethacin, indomethacin farnesil, acemetacin, proglumetacin maleate, amfenac sodium, nabumetone, ibuprofen, flurubiprofen, flurubiprofen axetil, ketoprofen, naproxen, protizinic acid, pranoprofen, fenoprofen calcium, tiaprofenic acid, oxaprozin, loxoprofen sodium, alminoprofen, zaltoprofen, phenylbutazone, clofezone, ketophenylbutazone, piroxicam, tenoxicam, ampiroxicam, tiaramide hydrochloride, tinoridine hydrochloride, benzydamine hydrochloride, epirizole and emorfazone.

As examples of the steroid agent, there can be cited cortisone acetate, hydrocortisone (phosphoester, acetate), hydrocortisone butyrate, hydrocortisone succinate sodium, prednisolone (acetate, succinate, tertiary butylacetate ester, phosphoester), methyprednisolone (acetate), methylprednisolone succinate sodium, triamcinolone, triamcinolone acetonide (triamcinolone acetate), dexamethasone (phosphoester, acetate, sodium phosphate, sulfuric acid ester), dexamethasone palmitate, betamethasone (phosphate, disodium salt), paramethasone acetate, fludrocortisone acetate, halopredone acetate, clobetasol propionate, halcinonide, beclomethasone propionate, betamethasone valerate, betamethasone acetate and cortisone acetate.

As examples of the angiotensin converting enzyme inhibitor, there can be cited alacepril, imidapril hydrochloride, temocapril hydrochloride, delapril hydrochloride, benazepuril hydrochloride, captopril, cilazapril, enalapril maleate and lisinopril.

As examples of the vasodilator, there can be cited theophylline, diprophylline, proxyphylline, aminophylline, choline theophylline, prostaglandin, prostaglandin derivatives, alprostadil alfadex, alprostadil, limaprost alfadex, papaverine, cyclandelate, cinnarizine, bencyclane fumarate, cinepazide maleate, dilazep hydrochloride, trapidil, difenidol hydrochloride, nicotinic acid, inositol hexanicotinate, nicametate citrate, nicotinic alcohol tartrate, tocopherol nicotinate, hepronicate, isoxsuprine hydochloride, bamethan sulfate, torarizone hydrochloride, dihydroergotoxine mesylate, ifenprodil tartrate, moxisylyte hydrochloride nicergoline; nicardipine hydrochloride, nilvadipine, nifedipine, benidipine hydrochloride, diltiazem hydrochloride, nisoldipine, nitrendipine, manidipine hydrochloride, barnidipine hydrochloride, efonidipine hydrochloride, verapamil hydrochloride, trimetazidine hydrochloride, captopril, enalapril maleate, alacepril, delapril hydrochloride, cilazapril, lisinopril, benazepuril hydrochloride, hydralazine hydrochloride, todralazine hydrochloride, budralazine, cadralazine, indapamide, carbochromen hydrochloride, efloxate, etafenone hydrochloride, oxyfedrine hydrochloride, nicorandil, amyl nitrite and isosorbide nitrate.

As examples of the growth/migration inhibitor for smooth muscle cells, there can be cited heparin sodium, dalteparin sodium (low molecular heparin), heparin calcium and dextran sulfate.

As examples of the platelet coagulation inhibitor, there can be cited ticlopidine hydrochloride, cilostazol, ethyl icosapentate, beraprost sodium, salpgrelate hydrochloride, batroxobin and dipyridamole.

As examples of the anticoagulant, there can be cited sodium heparin, sodium dalteparin (low molecular heparin), calcium heparin, dextran sulfate, warfarin potassium and argatroban.

As examples of the chemical mediator releasing inhibitor, there can be cited tranilast, ketotifen fumarate, azelastine hydrochloride, oxatomide, amlexanox and repirinast.

As examples of the immunosuppressor, there can be cited cyclosporine.

As examples of the antiviral agent, there can be cited aciclovir, ganciclovir, didanocine, zidovudine, sorivudine and vidarabine.

The kind of drug for diagnosis to be included is not particularly limited unless it does not harm the formation of drug carrier. Specifically, X-ray contrasting medium, radiolabeled nuclear medicine diagnostic drug and a nuclear magnetic resonance diagnostic drug for diagnosis.

For example, as examples of X-ray contrast medium, there can be cited amidotrizoate meglumine, iotalamate sodium, iotalamate meglumine, gastrographin, iodamide meglumine, lipiodol ultrafluid, adipiodone meglumine, ioxaglic acid, iotroxate meglumine, iotrolan, iopanoic acid, iopamidol, iohexol, ioversol, iopodate sodium, iomeprol, isopaque and iodoxamic acid.

The liposome of the present invention can be readily obtained by a common method. One example of such a method will be described below. Constituents such as a basic compound and an acidic compound, and other constituents such as a phospholipid, a stabilizer, and antioxidant are mixed in an organic solvent such as chloroform in a flask and the organic solvent is evaporated. Thereafter, drying under vacuum is performed to form a thin film on the inner wall of the flask. Then, a drug is added to the flask and vigorous stirring is performed, thereby obtaining a liposome dispersion. The obtained liposome dispersion is centrifuged and the supernatant is decanted to remove the drug that has not been encapsulated. Optionally, a lipid derivative solution of hydrophilic polymer may be further added as needed and the mixture is heated to obtain the liposome of the present invention.

The liposome of the present invention can be obtained also by adding a lipid derivative solution of hydrophilic polymer at the time of mixing the constituents. The method of adding the lipid derivative solution of hydrophilic polymer can be performed at any time without causing a particular problem. However, in the case of the method of adding it at the time of mixing the constituents, the lipid derivative of hydrophilic polymer is incorporated also in the inside of liposome and it may be sometimes the case that there occurs a substantial decrease in surface modification ratio and a decrease in encapsulated volume.

As an alternative method, the constituents may be mixed and the mixture may be discharged at a high pressure by use of a high pressure discharging type emulsifier to obtain the liposome of the present invention.

The method of administering the liposome of the present invention is not particularly limited and the administration is intravenous administration as a solution. The liposome of the present invention when administered to living organisms specifically accumulates in a region of acidic pH such as an inflammatory site or a tumor site, and in other regions, its accumulation is prevented. In consideration of this, it is suitable as a vehicle for anti-inflammatory agent or anti-tumor agent and has the effects of effectively causing the potency of the drugs to be exhibited and of alleviating their side effects.

Hereinafter, the present invention will be illustrated specifically along with examples.

### (Example 1)

### Preparation of liposome comprising 3,5-dipentadecyloxybenzamidine hydrochloride (basic compound) and myristic acid (fatty acid) as constituents

(1-[1]) First, a lipid mixed solution obtained by dissolving 4.62 mmol of phosphatidylcholine, 4.62 mmol of cholesterol, and 0.76 mmol of 3,5-dipentadecyloxybenzamidine hydrochloride in 100 ml of t-butyl alcohol was frozen in an ice bath and freeze-dried overnight to obtain a lipid mixture.
(1-[2]) 0.06 mmol of the lipid mixture obtained by the procedure in (1-[1]) was metered in a 50 ml eggplant type flask and 2 ml of a 1.14 mM myristic acid/chloroform solution was added thereto. After removal of the chloroform, the mixture was dried under vacuum overnight to form a lipid thin film on the inner wall of the flask.

To this, 6 ml of physiological saline was added and the mixture was subjected to ultrasonication at 55°C for 5 minutes to obtain a liposome dispersion. This was extruded under pressure by use of a polycarbonate film with a pore size of 0.4 µm, and then by use of a polycarbonate film with the pore size of 0.2 µm and a polycarbonate film with the pore size of 0.1 µm. Thus the entitled liposome dispersion having an average particle size of 90 to 200 nm was obtained. The entitled liposome dispersion comprising 7.3 mol% of the basic compound [1] of the present invention and 3.7 mol% of the acidic compound [2] of the present invention was obtained. The average particle size of the obtained liposome was 125.5 nm.

### (Example 2)

### Preparation of liposome comprising 3,5-dipentadecyloxybenzamidine hydrochloride (basic compound) and sodium prednisolone phosphate (phosphoric acid monoester derivative) as constituents.

To 0.6 mmol of the lipid mixture obtained by the procedure in (1-[1]) was added 10 ml of a 75 mM aqueous sodium prednisolone phosphate solution and the mixture was subjected to ultrasonication at 55°C for 5 minutes to obtain a liposome dispersion. This was subjected by particle size selection by feeding it under pressure by use of a polycarbonate film with a pore size of 0.4 µm, and then by use of a polycarbonate film with a pore size of 0.2 µm and a polycarbonate film with a pore size of 0.1 µm. The obtained liposome dispersion was applied to a column (Sepharose 4 Φ 3 cm × 20 cm, eluent: physiological saline) to collect liposome fraction to obtain the entitled liposome dispersion having a particle size of 90 to 200 nm (average particle size 130.7 nm).

### (Example 3)

### Preparation of liposome comprising 3,5-dipentadecyloxybenzamidine hydrochloride (basic compound) and sodium riboflavin phosphate (phosphoric acid monoester derivative) as constituents.

To 0.6 mmol of the lipid mixture obtained by the procedure in (1-[1]) was added 10 ml of a 75 mM aqueous sodium riboflavin phosphate solution and the mixture was subjected to ultrasonication at 55°C for 5 minutes to obtain a liposome dispersion. Subsequently, the same operation as in Example 2 was followed to obtain the entitled liposome dispersion. The average particle size of the obtained liposome was 117.9 nm.

### (Comparative Example 1)

To 0.6 mmol of the lipid mixture obtained by the procedure above-mentioned in (1-[1]) was added 10 ml of physiological saline and the mixture was subjected to ultrasonication at 55°C for 5 minutes to obtain a liposome dispersion. This was processed in the same manner as in (1-[2]) above to obtain the entitled liposome dispersion having an average particle size of 90 to 200 nm. The average particle size of the obtained liposome was 110.5 nm.

### (Comparative Example 2)

### Preparation of liposome comprising 3,5-dipentadecyloxybenzamidine hydrochloride and phosphatidylglycerol as constituents

0.06 mmol of the lipid mixture obtained by the procedure above-mentioned in (1-[1]) was metered in a 50 ml eggplant type flask and 2 ml of a 1.14 mM phosphatidylglycerol/chloroform solution was added thereto. After removal of the chloroform, the mixture was dried under vacuum overnight to form a lipid thin film on the inner wall of the flask. To this, 6 ml of physiological saline was added and the mixture was subjected to ultrasonication at 55°C for 5 minutes to obtain a liposome dispersion. This was processed in the same manner as in (1-[2]) above to obtain the entitled liposome dispersion having an average particle size of 90 to 200 nm. The average particle size of the obtained liposome was 138.8 nm.

### (Comparative Example 3)

### Preparation of liposome comprising 3,5-dipentadecyloxybenzamidine hydrochloride and polyethylene glycol-phosphatidylethanolamine derivative as constituents

The lipid concentration of the liposome dispersion obtained in Comparative Example 1 was adjusted to 20 mM. To this liposome dispersion was added same volume of polyethylene glycol- phosphatidylethanolamine derivative solution (molecular weight : about 6,000, and concentration : 1.214 mg/ml) and then the mixture was heated at 60°C for 30 minutes to obtain the entitled liposome dispersion having a particle size of 90 to 200 nm (average particle size : 117.4nm).

### (Test Example 1) Affinity of various kinds of liposomes for proteoglycan

The purpose of this test was to examine the affinity of liposome for proteoglycan overproduced at an inflammatory site or a tumor site under acidic condition and in the vicinity of neutrality.

### (Method)

Glycosaminoglycan serving as an anionic sugar chain polymer, contained in a proteoglycan was mixed with liposome in each of Examples and Comparative Examples and the amount of glycosamioglycan adsorbed on each liposome was measured. Chondroitin sulfate C (molecular weight : 30,000 to 60,000) as the glycosaminoglycan was dissolved in a phosphate buffer at pH 7.4 and adjusted to 0.1 mg/ml and further adjusted to pH 6.0 or pH 6.5 with 6N HCl. 0.3 ml of the obtained 0.1 mg/ml chondroitin sulfate C solution at pH 7.4 or pH 6.0 and 0.3 ml of each liposome dispersion of which the lipid concentration was adjusted to 10 mM with physiological saline were mixed and heated at 37°C for 1 hour. After cooling, ultracentrifugation was performed at 100,000 g for 1 hour and the supernatant was collected. Chondroitin sulfate C in the supernatant was determined by use of a Blyscan proteoglycan assay kit and the amount of adsorption of chondroitin sulfate C to each liposome was calculated.

### (Results)

The results are shown in Fig. 1. The liposomes in Comparative Example 1 in which no acidic compound was comprised and Comparative Examples 2 and 3 in which the acidic compound was a phosphodiester had the same adsorption amount of chondroitin sulfate C at pH 6.0 or 6.5, and pH 7.4. It was confirmed that the liposomes in accordance with Examples 1 to 3 of the present invention showed some difference in the adsorption amount for chondroitin sulfate C between pH 6.0 or 6.5 and pH 7.4 and at pH 7.4, the adsorption amount was suppressed. That is, it was observed that the liposomes of the present invention had suppressed affinity for proteoglycan in the vicinity of neutrality while in acidic environment it showed high affinity therefor.

### (Test Example 2) Acute Toxicity

The purpose of this test was to know the degree of toxicity of the liposome of the present invention as compared with the conventional ones. For this purpose, lethal toxicity tests of the liposome of the present invention on rats were performed.

### (Method)

The liposome dispersions of Examples 1 to 3 were concentrated by use of an ultrafiltration membrane and optionally diluted with sterilized distilled water for injection as needed to make test solutions. Quarantined 5-week aged mice were divided into groups of 5 animals per group and 100 ml/kg of the test solution was administered to mice via the tail vein. On the other hand, the solvent control group was administered with 100 ml/kg of physiological saline. After the administration of the test solution, the general condition was carefully observed at least once a day for 7 days and indication of toxicity and death case were recorded. Also, 7 days after administration, autopsy was performed to remove respective organs. Pathological sections were prepared for each organ and observation was performed thereon.

### (Results)

No death case was observed during the observation period in the case of the liposomes of the present invention. In the histopathological observation of each organ after 7 days, no histopathological change was observed. That is, it was confirmed that the liposomes of the present invention were extremely low in toxicity and high in safety.

### INDUSTRIAL APPLICABILITY

The liposomes of the present invention are very high in targeting capability at a diseased site such as inflammation and tumor where pH has been decreased in acidic regions. Also, they are very high in safety.

Therefore, the liposomes of the present invention that carry a drug for the diagnosis and/or therapy are very effective for the purpose of the diagnosis and therapy of tissues and/or organs where inflammation or tumor lesion has occurred.

## Claims

1. A liposome that includes a drug intended for the therapy and/or diagnosis, comprising as membrane constituents:
[1] a basic compound represented by the following formula 1 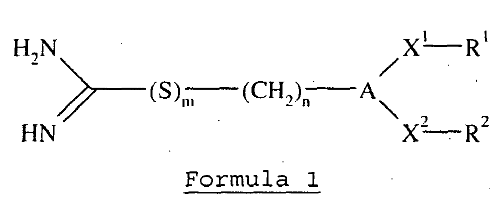 in which A represent an aromatic ring, R¹ and R² represent an alkyl group having 10 to 25 carbon atoms, where R¹ and R² may be the same or different, X¹ and X² represent -O-, -S-, -COO-, -OCO-, -CONH- or -NHCO, m is 0 or 1, n is 0 or an integer of 1 to 6; the molar ratio of the basic compound being 1 to 30 mol% of total liposome membrane constituents;
[2] an acidic compound which is a phosphoric acid monoester derivative, or a compound having a carboxyl group or its salt, said phosphoric acid monoester derivative being selected from prednisolone phosphate and riboflavin phosphate, and the molar ratio of said acidic compound being 0.5 to 30 mol% of total liposome membrane constituents; and
[3] a liposome membrane constituent other than [1] and [2].

2. The liposome according claim 1, wherein said compound having a carboxyl group or its salt, is a fatty acid.

3. The liposome according to claims 1 to 2, wherein said fatty acid is oleic acid, stearic acid, palmitic acid or myristic acid.

4. The liposome according to any one of claims 1 to 3, wherein the liposome membrane constituent other than [1] and [2], is phospholipid and/or sterol.

5. The liposome according to any one of claims 1 to 4, wherein said drug intended for the therapy and/or diagnosis is an anti-cancer agent, an antibiotic, an enzyme agent, an enzyme inhibitor, an antioxidant, a lipid uptake inhibitor, a hormone agent, an anti-inflammatory agent, a steroid agent, a vasodilator, an angiotensin converting enzyme inhibitor, an angiotensin receptor antagonist, a growth/migration inhibitor for smooth muscle cells, a platelet aggregation inhibitor, an anticoagulant, a chemical mediator releasing inhibitor, a vascular endothelial cell growth or suppressing agent, an aldose reductase inhibitor, a mesangium cell growth inhibitor, a lipoxygenase inhibitor, an immunosuppressor, an immunoactivator, an antiviral agent, a Maillard reaction inhibitor, an amyloidosis inhibitor, an NOS inhibitor, an AGEs inhibitor, or a radical scavenger.

6. The liposome according to any one of claims 1 to 5, wherein said drug intended for the therapy and/or diagnosis is a nucleic acid, polynucleotide or a gene.

7. The liposome according to any one of claims 1 to 6, wherein said drug intended for the therapy and/or diagnosis is a glycosaminoglycan.

8. The liposome according to any one of claims 1 to 7, wherein said drug intended for the therapy and/or diagnosis is oligo- and/or polysaccharide.

9. The liposome according to any one of claims 1 to 8, wherein said drug intended for the therapy and/or diagnosis is protein or peptide.

10. The liposome according to any one of claims 1 to 9, wherein said drug intended for the therapy and/or diagnosis is an intracorporeal diagnostic drug such as an X-ray contrasting medium, a radiolabeled nuclear medicinal diagnostic drug, or a nuclear magnetic resonance diagnostic drug for diagnosis.

## Patentansprüche

1. Liposom, das ein Arzneimittel enthält, das für die Therapie und/oder Diagnose bestimmt ist, umfassend als Membranbestandteile:
[1] eine Basisverbindung, die durch die folgende Formel 1 dargestellt ist: 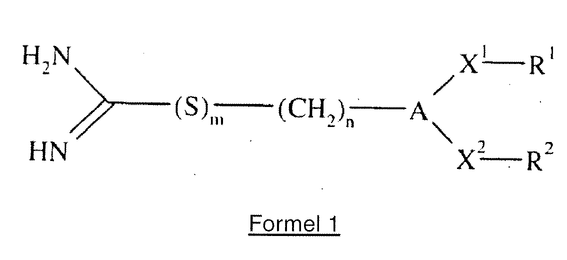 wobei A einen aromatischen Ring darstellt, R¹ und R² eine Alkylgruppe mit 10 bis 25 Kohlenstoffatomen darstellen, wobei R¹ und R² gleich oder verschieden sein können, X¹ und X² -O-, -S-, -COO-, -OCO-, -CONH- oder -NHCO- darstellen, m 0 oder 1 ist, n 0 oder eine ganze Zahl von 1 bis 6 ist; wobei das Molverhältnis der Basisverbindung 1 bis 30 Mol% der gesamten Liposommembranbestandteile ist,
[2] eine saure Verbindung, die ein Phosphorsäuremonoesterderivat oder eine Verbindung mit einer Carboxylgruppe oder deren Salz ist; wobei das Phosphorsäuremonoesterderivat ausgewählt ist aus Prednisolonphosphat und Riboflavinphosphat, und das Molverhältnis der sauren Verbindung 0,5 bis 30 Mol% der gesamten Liposommembranbestandteile beträgt, und
[3] einen Liposommembranbestandteil, der von [1] und [2] verschieden ist.

2. Liposom nach Anspruch 1, wobei die Verbindung mit einer Carboxylgruppe oder deren Salz eine Fettsäure ist.

3. Liposom nach Anspruch 1 bis 2, wobei die Fettsäure Oleinsäure, Stearinsäure, Palmitinsäure oder Myristinsäure ist.

4. Liposom nach einem der Ansprüche 1 bis 3, wobei der Liposommembranbestandteil, der von [1] und [2] verschieden ist, Phospholipid und/oder Sterol ist.

5. Liposom nach einem der Ansprüche 1 bis 4, wobei das Arzneimittel, das für die Therapie und/oder Diagnose bestimmt ist, ein Anti-Krebsmittel, ein Antibiotikum, ein Enzymagens, ein Enzyminhibitor, ein Antioxidans, ein Lipidaufnahme-Inhibitor, ein Hormonagens, ein entzündungshemmendes Mittel, ein Steroidagens, ein Vasodilator, ein Angiotensinumsetzungsenzym-Inhibitor, ein Angiotensinrezeptor-Antagonist, ein Wachstums-/Migrationsinhibitor für glatte Muskelzellen, ein Plättchenaggregations-Inhibitor, ein Antikoagulans, ein Inhibitor für die Freisetzung eines chemischen Mediators, ein Wachstums- oder Unterdrückungsagens für vaskuläre Endothelzellen, ein Aldose-Reductase-Inhibitor, ein Mesangium-Zellwachstumsinhibitor, ein Lipoxygenase-Inhibitor, ein Immunsuppressor, ein Immunaktivator, ein antivirales Agens, ein Maillard-Reaktions-Inhibitor, ein Amyloidose-Inhibitor, ein NOS-Inhibitor, ein AGEs-Inhibitor oder ein Radikalfänger ist.

6. Liposom nach einem der Ansprüche 1 bis 5, wobei das Arzneimittel, das für die Therapie und/oder Diagnose bestimmt ist, eine Nukleinsäure, ein Polynukleotid oder ein Gen ist.

7. Liposom nach einem der Ansprüche 1 bis 6, wobei das Arzneimittel, das für die Therapie und/oder Diagnose bestimmt ist, ein Glycosaminoglycan ist.

8. Liposom nach einem der Ansprüche 1 bis 7, wobei das Arzneimittel, das für die Therapie und/oder Diagnose bestimmt ist, ein Oligo- und/oder Polysaccharid ist.

9. Liposom nach einem der Ansprüche 1 bis 8, wobei das Arzneimittel, das für die Therapie und/oder Diagnose bestimmt ist, ein Protein oder Peptid ist.

10. Liposom nach einem der Ansprüche 1 bis 9, wobei das Arzneimittel, das für die Therapie und/oder Diagnose bestimmt ist, ein intrakorporales diagnostisches Arzneimittel ist, wie ein Röntgenkontrastmittel, ein radiomarkiertes, nukleares, medizinisches, diagnostisches Arzneimittel oder ein nukleares, diagnostisches Magnetresonanz-Arzneimittel zur Diagnose ist.

## Revendications

1. Liposome qui comprend un médicament prévu pour le traitement et/ou le diagnostic, comprenant, comme constituants membranaires :
(1) un composé basique représenté par la formule 1 suivante : 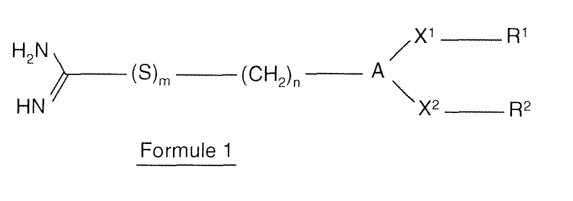 dans laquelle A représente un cycle aromatique, R¹ et R² représentent un groupe alkyle ayant de 10 à 25 atomes de carbone, où R1 et R² peuvent être identiques ou différents, X¹ et X² représentent -O-, -S-, -COO-, -OCO-, -CONH- ou -NHCO, m est 0 ou 1, n est 0 ou un entier de 1 à 6 ; le rapport molaire du composé basique étant de 1 à 30 % en mole des constituants membranaires totaux du liposome ;
(2) un composé acide qui est un dérivé monoester de l'acide phosphorique ou un composé ayant un groupe carboxyle ou son sel, ledit dérivé monoester de l'acide phosphorique étant sélectionné parmi le phosphate de prednisolone et le phosphate de riboflavine, et le rapport molaire dudit composé acide étant de 0,5 à 30 % en mole des constituants membranaires totaux du liposome ; et
(3) un constituant membranaire du liposome autre que (1) et (2).

2. Liposome selon la revendication 1, où ledit composé ayant un groupe carboxyle ou son sel est un acide gras.

3. Liposome selon les revendications 1 et 2, où ledit acide gras est l'acide oléique, l'acide stéarique, l'acide palmitique ou l'acide myristique.

4. Liposome selon l'une quelconque des revendications 1 à 3, où le constituant membranaire du liposome autre que (1) et (2) est un phospholipide et/ou un stérol.

5. Liposome selon l'une quelconque des revendications 1 à 4, ou ledit médicament prévu pour le traitement et/ou le diagnostic est un agent anticancéreux, un antibiotique, un agent enzymatique, un inhibiteur enzymatique, un antioxydant, un inhibiteur de l'absorption de lipides, un agent hormonal, un agent anti-inflammatoire, un agent stéroïdien, un vasodilatateur, un inhibiteur de l'enzyme de conversion de l'angiotensine, un antagoniste des récepteurs de l'angiotensine, un inhibiteur de la croissance/migration des cellules musculaires lisses, un antiagrégant plaquettaire, un anticoagulant, un inhibiteur de la libération de médiateurs chimiques, un agent de croissance ou de suppression des cellules vasculaires endothéliales, un inhibiteur de l'aldose-réductase, un inhibiteur de la croissance des cellules mésangiales, un inhibiteur de la lipo-oxygénase, un immunosuppresseur, un immunoactivateur, un agent antiviral, un inhibiteur de la réaction de Maillard, un inhibiteur de l'amyloïdose, un inhibiteur non spécifié, un inhibiteur des produits terminaux de la glycolysation avancée, ou un antiradicalaire.

6. Liposome selon l'une quelconque des revendications 1 à 5, où ledit médicament prévu pour le traitement et/ou le diagnostic est un acide nucléique, un polynucléotide ou un gène.

7. Liposome selon l'une quelconque des revendications 1 à 6, où ledit médicament prévu pour le traitement et/ou le diagnostic est un glycosaminoglycane.

8. Liposome selon l'une quelconque des revendications 1 à 7, où ledit médicament prévu pour le traitement et/ou le diagnostic est un oligosaccharide et/ou un polysaccharide.

9. Liposome selon l'une quelconque des revendications 1 à 8, où ledit médicament prévu pour le traitement et/ou le diagnostic est une protéine ou un peptide.

10. Liposome selon l'une quelconque des revendications 1 à 9, où ledit médicament prévu pour le traitement et/ou le diagnostic est un médicament de diagnostic intracorporel tel qu'un radio-opacifiant, un médicament de diagnostic de médecine nucléaire radiomarqué ou un médicament de diagnostic par résonance magnétique nucléaire pour le diagnostic.
